# EUROPEAN PATENT SPECIFICATION

(11) **EP 3 195 166 B1**
(45) Date of publication and mention of the grant of the patent: **21.08.2024**
(21) Application number: 15841896.2
(22) Date of filing: 14.09.2015
(51) Int. Cl.: G06Q 10/087, G16H 40/20

(54) **INSTRUMENT MANAGEMENT SYSTEM**
INSTRUMENTENVERWALTUNGSSYSTEM
SYSTÈME DE GESTION D'INSTRUMENT

(30) Priority: 15.09.2014 US 201462050525 P
(43) Date of publication of application: 26.07.2017
(73) Proprietor: Leica Biosystems Melbourne Pty Ltd, Mt Waverley, VIC 3149 (AU)
(72) Inventor: QERIM, Shahini, Bentleigh East, Victoria 3165 (AU); CHESWORTH, Iain, Sandringham, Victoria 3191 (AU); MADDERN, James, Mont Albert, Victoria 3127 (AU); LITOW, Micah, Chicago, Illinois 60614 (US)
(74) Representative: Dehns Germany Partnerschaft mbB
(86) International application number: PCT/AU2015/000566
(87) International publication number: WO 2016/040985

(56) References cited:
- EP-A1- 2 528 026
- WO-A1-2015/013688
- US-A1- 2010 292 556
- US-A1- 2011 270 711
- US-A1- 2012 179 585
- US-A1- 2012 299 928
- US-A1- 2013 138 452
- ANONYMOUS: "Data conversion - Wikipedia", 8 September 2014 (2014-09-08), XP055450454, Retrieved from the Internet <URL:https://en.wikipedia.org/w/index.php?title=Data_conversion&oldid=624635283> [retrieved on 20180212]

## Description

### Technical Field

The present invention relates to systems for controlling, managing and enhancing operation of devices. It relates particularly, but not exclusively, to systems that are operable with mobile and other computerised devices, to access data and control signals from laboratory devices namely, instrumentation and related devices used in the processing of tissue and other specimens for pathology testing and the like.

### Background of Invention

Laboratory instruments are used to perform specimen processing, typically for diagnostic purposes. They are often high cost instruments using high value reagents and other consumables. The tests that they perform often can be critically important in the diagnosis and effective treatment of disease and disorders, but they are often expensive. Errors in processing, delays and instrument down time can add to the cost of operating laboratories in which these instruments are used. US 2012/0179585 A1 discloses a method and system for identification and inventory management for laboratory assets, particularly to the tracking of location, status, and/or use of equipment and consumable items used in laboratory research. A method is provided for receiving information from an e.g. RFID tag, barcode or a sensor, which information is related to a location, a status or a use for each of a plurality of research assets. The received information is added to a database, and the database can be accessed to identify a portion of the research assets in need of an action. The information related to the research assets may be bundled by the system to provide downstream outputs to an end user or a report related to a research asset is generated by the system and provided to an end user. US 2012/0299928 A1 relates to graphically based methods for displaying the status of an instrument in a laboratory and quality control results obtained from an instrument in a laboratory. EP 2528026 A1 relates to a system and method of inventory management, wherein a plurality of client devices and a clinical analyser are connected to a network, which is connected to a server.

Most modern laboratory instruments are automated in some way and computer control is becoming commonplace. Nevertheless, each instrument may have a unique operating system and use different data schema and control signals. This is beneficial in that advancement is not limited by a requirement to adhere to standards that may be outdated. However, it inhibits integrated management of multiple instruments. This problem is exacerbated in laboratories containing instruments from different manufacturers and of significantly different age.

It would be desirable to provide a platform or system that permits integrated and streamlined management of multiple instruments in a laboratory. It would also be desirable to provide a system that provides users with customised interaction with an instrument in a laboratory.

The discussion of the background to the invention included herein including reference to documents, acts, materials, devices, articles and the like is included to explain the context of the present invention. This is not to be taken as an admission or a suggestion that any of the material referred to was published, known or part of the common general knowledge in Australia or in any other country as at the priority date of any of the claims.

### Summary of Invention

Viewed from one aspect, the present invention provides a system for customising management of one or more instruments in a laboratory according to claim 1. The system includes: a user device adapted to receive inputs from a user, the user device having a device processor for processing the received inputs and instrument data from the one or more instruments, and a device display for displaying instrument information; an interface module communicatively coupled with the user device and the one or more instruments and configured to convert instrument data in a first format generated by an instrument processor to a second format for processing by the device processor; and an instrument module configured to cause the device processor to provide customised instrument information on the device display.

Preferably, the user device is configurable with an instrument module by: a user requesting application software corresponding to the instrument module from a remotely located vendor; the vendor approving the request and operating a distribution software program to deliver the requested application software to the user device; and the user installing the application software on the user device to activate the instrument module. Typically this is enabled in a cloud computing environment.

The instrument module is selectable from a suite of instrument modules with which the user device may be configured including an inventory management module, and preferably but not limited to: an instrument management module; a statistics module; a workflow module; a maintenance module; and a reporting module.

An inventory management module may be configurable for customised inventory management by performing functions on the device processor selected from the group including: interrogating for and/or receiving automatically from one or more instruments inventory levels for individual ones of the instruments; interrogating for and/or receiving automatically from instruments in the laboratory aggregated inventory levels across a plurality of instruments in the laboratory; interrogating for and/or receiving automatically from one or more instruments in the laboratory an estimated time at which supply of a consumable in the laboratory will be critically low; automatic ordering of one or more consumables used by an instrument in the laboratory; tracking an order status for consumables ordered by the inventory management module; presenting on the device display inventory management information; and automatically invoicing an entity for consumables ordered by the inventory management module.

Additionally, the inventory management module is configured to determine automatically when the user device is in close physical proximity to an instrument in the laboratory that has an inventory management issue and automatically issue a notification to the user device advising the operator to perform an inventory management operation on the instrument.

An instrument management module is configurable for customised instrument management by performing functions on the device processor selected from the group including: interrogating for and/or receiving automatically instrument status information from one or more instruments and presenting on the device display status information for the one or more instruments; interrogating for and/or receiving automatically alert data from one or more instruments and presenting on the device display an alert symbol or notification, for respective ones of the instruments; interrogating for and/or receiving automatically an urgent specimen signal and presenting on the device display an urgency symbol or notification for one or more of the instruments associated with the urgent specimen signal; receiving from the user customisation rules, and customising the instrument management module according to the customisation rules. Typically, the instrument status is represented by an icon.

The customisation rules may include e.g. user-defined instrument naming, user-defined instrument positioning on the display, user-defined instrument parameters for presentation on the display and user-defined instrument control features controllable by the user device using the instrument management module to name a few.

A workflow module may be configurable for customised management of specimen processing steps performed by one or more instruments in the laboratory, by performing functions on the device processor selected from the group including: receiving electronically a request for a specimen processing step not already scheduled on an instrument and causing scheduling of the requested processing step on an instrument. Ideally, the scheduling performed by the workflow module optimises use of instruments, and timing of processing steps, across instruments in the laboratory. The workflow module may include further functions such as receiving specimen data from one or more instruments and presenting on the device display a related image or result of a processing step; processing specimen data received from one or more instruments and automatically presenting on the device display a recommended action; receiving specimen processing schedules from a plurality of instruments and automatically re-scheduling one or more processing steps to optimise one or more constraints selected from the group including scheduled completion time, consumables used, operational cost, staffing requirements and specimen processing value; and presenting current status information for specimen processing steps.

A maintenance module may be configurable for customised instrument maintenance by performing functions on the device processor selected from the group including: interrogating for and/or receiving automatically maintenance data from one or more instruments in the laboratory and causing the user device to display a prompt when ones of the instruments have a scheduled maintenance event; interrogating and/or receiving automatically aggregated maintenance data from a plurality of laboratories and adjusting a scheduled maintenance event based on the aggregated data; aggregating scheduled maintenance events for a plurality of instruments in the laboratory and creating new maintenance schedule which optimises one or more constraints selected from the group including scheduled instrument downtime, technician availability, and impact on scheduled or expected specimen processing; and transmitting to a remote processing device maintenance data from the one or more instruments for aggregation with maintenance data from one or more other laboratories.

A reporting module may be configurable for customised reporting of instrument related performance data, by performing functions on the device processor selected from the group including: receiving operator defined specifications for data analysis; receiving operator defined specifications for data reporting; aggregating instrument performance data for a plurality of instruments in the laboratory and presenting on the device display laboratory performance information based on the aggregated instrument performance data; and transmitting to a remote processing device instrument and/or laboratory specific performance data for aggregation with performance data from one or more other laboratories.

The user device may be a handheld device such as a smart phone or tablet computer although a notebook computer, netbook computer, body-worn computer (including devices such as Google^{™} glasses, smartwatches, virtual reality goggles and the like or various other mobile computing devices are contemplated. Non-mobile computing devices may also be suitable for achieving user device functionality.

In some embodiments, the instrument module is configurable to determine automatically when the user device is in close physical proximity to an instrument in the laboratory; and automatically issue a notification to the user device which is relevant to an instrument in close proximity. Similarly, the instrument module may be configurable in accordance with customisation rules received from the user as inputs to the user device, to customise the user's interaction with instruments through the user device.

Viewed from another non-claimed aspect, the present application discloses a method of deploying a platform for integrated management of instruments in a laboratory, the platform including one or more instrument modules deployable to user devices operated by users, the method including the steps of: a user operating a user device to request application software corresponding to an instrument module from a remotely located vendor; the vendor approving the request and operating a distribution software program to deliver the requested application software to the user device; and installing the application software on the user device to activate the requested instrument module.

Typically, after approval of a first request associated with a laboratory, the method includes causing installation of an interface module in the laboratory to permit transmission of laboratory instrument information from the instruments to the user device.

### Brief Description of Drawings

Figure 1 is a schematic illustration of a system for customising management of one or more instruments in a laboratory, according to an embodiment of the invention.
Figure 2 is a schematic illustration of a user device according to an embodiment of the invention.
Figure 3 shows a schematic illustration of an instrument communicatively coupled with an interface module according to an embodiment of the invention.
Figure 4 is a flow diagram illustrating a method for deploying a platform for integrated management of instruments in a laboratory, according to an embodiment of the invention.
Figure 5 is an example of a display interface for providing inventory information to a user according to an embodiment of the invention.
Figure 6 is an example of an order status screen for providing order status information to a user according to an embodiment of the invention.
Figure 7 is an example of a display interface for providing instrument management information to a user according to an embodiment of the invention.
Figure 8 is an example of a statistics screen for providing instrument statistics to a user according to an embodiment of the invention.
Figure 9 is an example of test information that may be made available by a workflow module according to an embodiment of the invention.

### Detailed Description

The present invention will now be described in greater detail with reference to the accompanying drawings. It is to be understood that the embodiments shown are examples only and are not to be taken as limiting the scope of the invention as defined in the claims appended hereto.

Where the terms "comprise", "comprises", "comprised" or "comprising" are used in this specification (including the claims) they are to be interpreted as specifying the presence of the stated features, integers, steps or components, but not precluding the presence of one or more other features, integers, steps or components or group thereof.

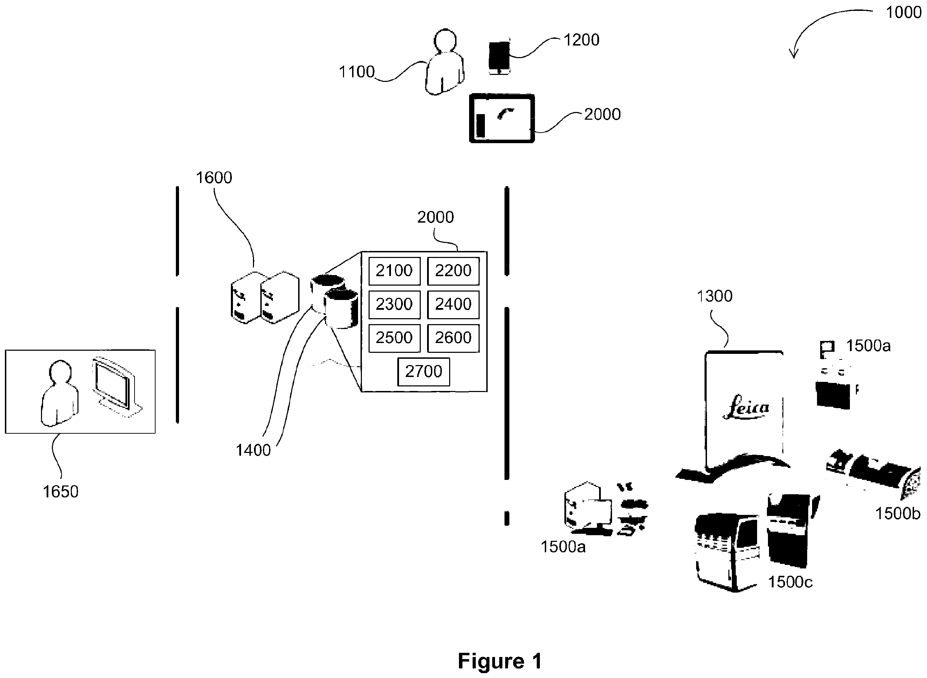

Referring firstly to Figure 1, there is shown a system 1000 for custom ising management of one or more instruments 1500a-d. Typically, instruments 1500a-d are located in a laboratory. Although they may be located in the same physical laboratory space, it is to be understood that a "laboratory" in which the instruments 1500 are located may be a virtual laboratory in that the instruments share a common owner or operator but they need not be in the same physical location or share the same manufacturer.

A user 1100 may interact with instruments 1500 by controlling a user device 1200 as required. Typically, the user device 1200 is a handheld or mobile computing device such as a smartphone, tablet or laptop computer, desktop computer or the like although it is to be understood that the functionality of user device 1200 may be incorporated into any suitable appliance capable of at least the user device functionality as described herein. For simplicity, such devices are referred to generically as "user device" 1200.

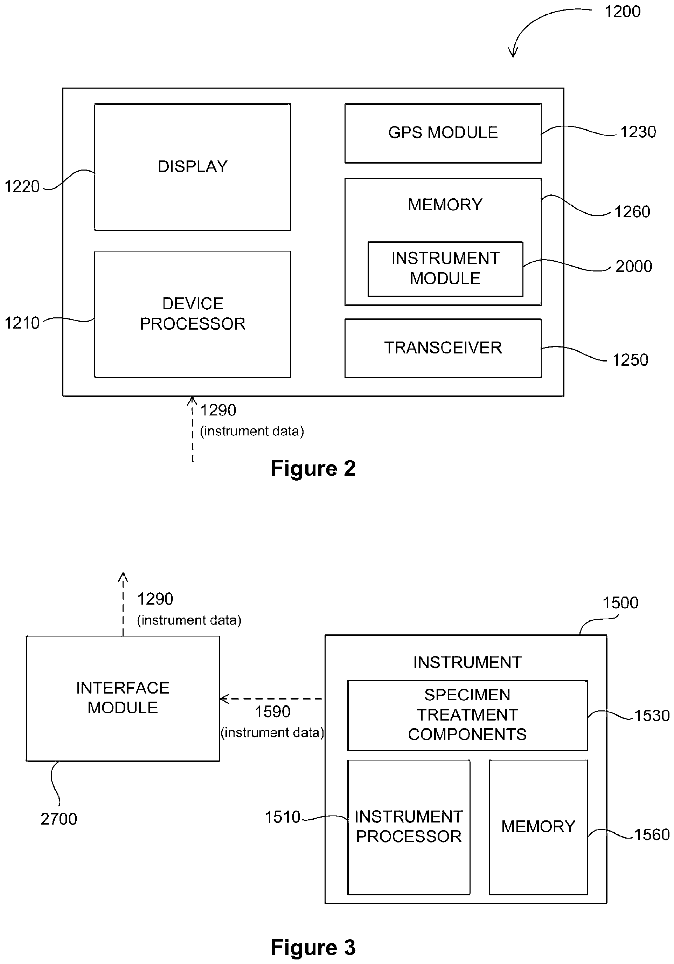

A schematic illustration of components of a typical user device 1200 is shown in Figure 2; a schematic illustration representing components of an instrument 1500 is shown in Figure 3. User device 1200 receives inputs from user 1100 e.g. by a touch sensitive display, a stylus, keyboard, voice command or the like. A device processor 1210 processes the received inputs as well as instrument data 1290 received from instruments 1500 via a transmitter (e.g. transmitter/receiver) 1250. A display 1220 on the display device 1200 presents to the user 1100 instrument information in a format which facilitates management of instruments 1500 in the laboratory. Advantageously, the user's interaction with the instruments may be customised by instrument module 2000 which causes the device processor 1210 to provide instrument information on the device display 1220 in a display format which is meaningful to the user. Various non-limiting examples of instrument modules are provided herein although one of skill in the art would appreciate, having regard to the examples provided, that various other iterations and combinations of display elements and parameters, arrangements of information and the like for customised interaction with instruments may be possible and may be brought into effect by the instrument module according to broad aspects of the present invention.

System 1000 has an interface module 2700 which is communicatively coupled (through a communications infrastructure such as the internet, Wi-Fi or other communication network, Bluetooth, RFID, cellular and others) with user device 1200 and the one or more instruments 1500. Typically, the interface module 2700 is provided on a secure computing device 1300 located in the laboratory. Alternatively, the functionality of interface module 2700 may be incorporated into a remote processor located "in the cloud" (as shown in Figure 1). It receives data from instruments 1500 and converts the instrument data from a first format 1590 generated by a processor 1510 in the instrument 1500 to a second format 1290 for processing by the device processor 1210. Instrument data may include any data that is collected by the instrument, such as reagent volumes, start and expiry dates, schedules for specimen treatment steps to be performed by the instrument, instrument installation, service and log data, instrument error data, and specimen data including test type, patient, clinician, hospital and batch data to name a few. Data may also be stored by instrument memory 1560 which also stores instructions for controlling one or more specimen treatment components 1530 of the instrument 1500.

The first data format 1590 produced by instruments 1500 may be of a schema or type which is common to all instruments in the laboratory (but not common to user device 1200) although that may not always be the case. The format of the instrument data may depend on the make, model, age and/or type of instrument. However the interface module 2700 is configurable to receive instrument data 1590 in the format it is created by the instrument, and convert it to a second format 1290 which is receivable and understood by an instrument module 2000 installed on the user device 1200. In another embodiment, the functionality of interface module 2700 may be brought into effect in software operating on one or more of the instruments 1500 and/or on the user device 1200 adapted to receive instrument data in a first format 1590 and convert and transmit instrument data in a second format 1290, via a communications infrastructure, to one or more user devices 1200.

An advantage of embodying interface module 2700 on secure computing device 1300 located in a laboratory, is that critical instrument data conversions are secure. Additional functionality relating to patient data security may also be incorporated into the secure computing device 1300 which may further be configured to encrypt patient data for transmission to the user device 1200 for use by particular instrument modules 2000.

System 1000 includes an instrument module 2000 which causes the device processor 1210 to provide, among other things, customised instrument information on the device display 1220. In Figure 1 instrument module 2000 is represented by a graphical display image although in reality, it is comprised of software stored in memory 1260 of display device 1200 (or in the cloud) that is configured to cause the device processor 1210 to perform processing functions that provide the user with a customised environment for interacting with instruments 1500 in the laboratory. Different instrument modules 2000 having different functionality may be installed on user device 1200.

In one example, to obtain access to an instrument module, a user 1100 instigates a process of selecting and purchasing application software for installation on user device 1200. This is similar to purchasing an "app" from online marketplaces such as Google Play Store, Apple App Store and Windows Phone Marketplace, to install functionality on an Android phone or tablet, iPhone or iPad, Windows phone or tablet, a body wearable device including computer glasses, virtual reality headset, or similar device.

In non-claimed examples, a user 1100 requests application software1400 corresponding to an instrument module 2000 from a remotely located vendor 1600. In one embodiment, vendor 1600 approves the request and operates a distribution software program (akin to those used by online market places as referred to above) to deliver the requested application software 1400 to the user device 1200. Approval may be contingent upon the user fulfilling an event, for example making payment or entering into some other arrangement with vendor, for the requested instrument module 2000. User 1100 installs the application software 1400 on the user device1200 to activate the instrument module 2000. In another embodiment, user 1100 can download the application software 1400 corresponding to an instrument module 2000 without prior approval of the request from the vendor 1600. However, the application software 1400 will remain inactive until such time that vendor approval is sought and obtained by the user 1100.

Typically, the process also includes, at least for an initial instrument module purchase for access to an instrument 1500 in a laboratory, the vendor instigating a process for shipment of an interface module 1300 to the laboratory and arranging for a service technician to install the interface module in the laboratory. Alternatively, the system may be provided as a plug and play system, or a combination of plug and play and service technician installation. Installation may be in relation to a hardware or a software version of the interface module 1300 which, once installed, provides the necessary interface and data conversion functionality between instruments 1500 and user device 1200. In some examples, a laboratory may authorise access by more than one user device 1200, to instruments 1500 using the interface module 1300. Typically, each user 1100 of a device 1200 requests and installs application software 1400 for the installation of an instrument module on that device although it is to be understood that some devices may be sold or offered to some users with one or more instrument modules pre-installed.

Various instrument modules according to embodiments of the present invention will now be described. These may include, by way of example, an inventory management module 2100, an instrument management module 2200, a statistics module 2300, a workflow module 2400, a maintenance module 2500 and a reporting module 2600. Other examples of instrument modules that may be installed on the display device include but are not limited to:
- a protocol advisor module that allows a user to find or access a recommendation for e.g. staining or other specimen treatment protocols, trouble shooting protocols or guidance for dealing with difficulties in tissue staining. A protocol advisor module may also rank or comment on usefulness of recommendations or allow the user to rank or comment by providing an input using the user device;
- a protocol optimiser module that enables a user to take an existing protocol and improve it or adapt it to another laboratory, user, instrument type, same instrument type from a different manufacturer, new version instrument or the like;
- an automatic workflow manager module which indicates which instrument can process a slide in a required time frame;
- a time manager module that assists with organising usage of instruments during the course of a day (or a shift, week, month etc.) to optimise instrument usage based on scheduled protocols e.g. to finish staining tasks on time or before the end of the day or other scheduled deadline;
- a maintenance module that displays information such as a summary of maintenance activities that are due (and/or overdue, approaching due), recently completed maintenance, estimated maintenance needs for the laboratory, lot numbers and the like, schedules maintenance automatically including booking a service call and generating a list of required parts, health and usage monitoring (HUMS), i.e. preventative and predictive maintenance based on analytics of an instruments operational performance/parameters. A maintenance module is operable across instruments types and manufacturers and supports configuration management;
- a test/slide tracking module which uses data collected by a barcode (or similar) reader on an instrument to provide user access via the user device 1200 to all information available concerning a barcoded (or other uniquely identifiable) slide, test, reagent, instrument operator and the like. The test/slide tracking module can recognise/capture a barcode and correlate subsequent events associated with that barcode;
- a test order module which enables pathologists or others to place orders for all types of tests that the laboratory can perform, and provides test status updates on a push or pull basis (real time enabled) including test results;
- an image bank module which provides users with access to features such as control or reference slide images for use in analysis or review of results, and for accessing databases of images from other resources for enhanced evaluation of test results by a user;
- a reference module which provides up to date access, on the user device, of latest news, antibody panels recommended for diseases, antibodies shown with supporting images and data for ongoing development of the user's knowledge. Automated tracking of reviewed material by the reference module may be utilised by to update professional development or ongoing education records. The reference module may provide guidance on the desired level of billing. For example, the module may provide an antibody panel recommendation based on desired cost of test;
- a capacity manager module tracks utilisation of instruments and operators in laboratories to monitor performance of staff and capacity of instruments, provides processing capacity forecasts and integrates with personnel management systems to manage availability of operators to oversee instruments in the laboratory, and minimise instrument downtime.

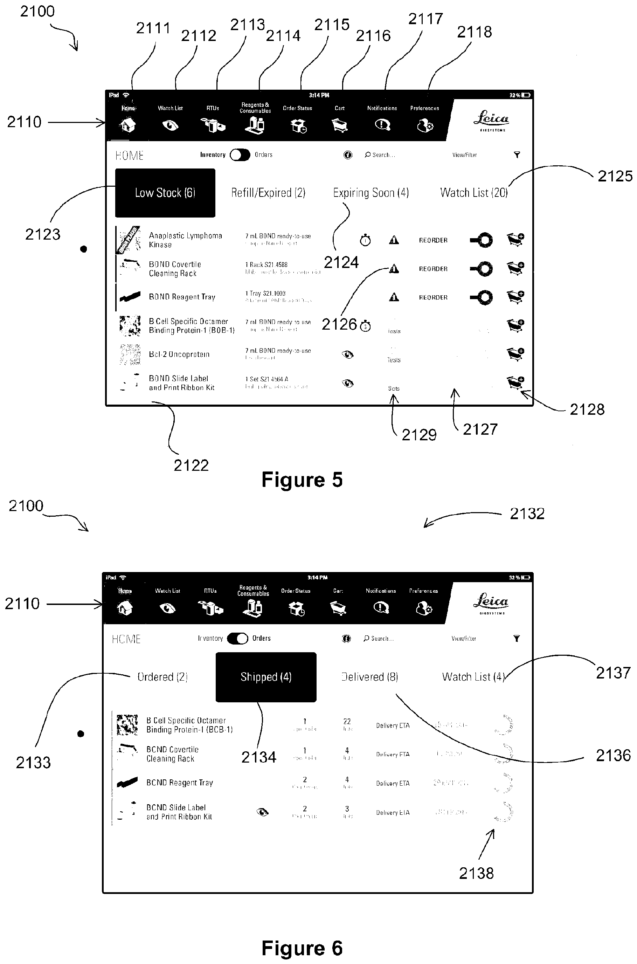

An inventory management module 2100 is configured for customised management of inventory such as reagents and other consumables (such as covertiles and coverslips) used by instruments in the laboratory. Figure 5 is an example of a display interface for a user device 1200 which has several display zones for providing inventory information to the user 1100. Menu zone 2110 includes icons that are selectable by a user to perform various inventory management functions. A "home" icon 2111 as is customary in software interfaces is provided to return the user to a "home" screen from which any functionality can be accessed directly or indirectly. In some embodiments, the user can configure the home screen that is accessed by selecting home icon 2111.

An example of a Home screen 2122 for an inventory management module 2100 according to an embodiment of the present invention is presented in Figure 5. The screen is divided into sections or 'tabs' 2123, 2124, 2125 that can be selected by the user to change the view of the screen. Selection of tab 2123 presents a list of consumables for which there is little or no stock remaining in the laboratory. Graphical symbols may be used to enhance user experience and richness of visual information provided through the interface. For example, warning symbol 2126 indicates when a particular product (e.g. Anaplastic Lymphoma Kinase, BOND Covertile cleaning Rack and Bond Reagent Tray) is out of stock. Supply symbol 2127 indicates the amount of a product remaining in the laboratory, where the symbol may be colour coded e.g. red when out of stock, and orange when stock is low (e.g. B Cell Specific Octamer Binding Protein-1, Bcl-2 Oncoprotein, BOND Slide Label and Print Ribbon Kit). Tests Remaining indicator 2129 shows a value calculated by the inventory management module to represent the number of tests that may be performed by the instruments, based on current inventory supplies, before they are exhausted. Cart symbol 2128 may be selected for an associated product to place an order for that product.

A 'cart' screen (not shown) may be viewed by selecting Cart icon 2116. When the order is finalised, a product order is transmitted, by inventory management module 2100, from user device 1200 to the product supplier. The order can be tracked by selecting Order Status tab 2115.

Selection of tab 2124 presents a list of consumables that require refilling (i.e. are empty) or have expired (past their useful date). Selection of tab 2125 presents a watchlist of products that the user wishes to track. The watchlist may be configured by the user and/or populated automatically, at least in part. For example, when the user orders a product that is 'out of stock' with the supplier, that product is automatically added to the watchlist. The watchlist may also be viewed by selecting Watchlist icon 2112 in menu zone 2110.

Also in menu zone 2110 is any inventory item, reagent or consumable that is automatically tracked by a connected instrument, for example, the RTU icon 2113 which takes the user to a filtered view of RTU reagent status only. Inventory items, reagents or consumables include items such as covertiles, coverslips, cleaning kits, detection systems and theranostic devices (e.g. Leica BOND Oracle). Use of covertiles may be tracked using a usage chip applied to the covertile. Reagents are tracked by a unique product identifier. A Reagents & Consumables icon 2114 takes the user to a filtered view of non-RTU consumables and reagents status only including anything that is not automatically tracked such as individual items in boxes of gloves, boxes of slide, tubes, and the like which are tracked at supply order level rather that individual item. An Order Status icon 2115 takes the user to a screen where the user can view the status of orders placed through the inventory management module (or using other ordering processes). The Order Status screen may be embodied in a number of different formats, where orders may be viewed at the aggregate level, or at the individual product level.

An example of an Order Status screen 2132 for an inventory management module 2100 according to an embodiment of the present invention is presented in Figure 6. The screen may be divided into sections or tabs that can be selected by the user to change the view on the screen or the data displayed on the screen. Selection of tab 2133 presents a list of consumables that have been ordered but not yet dispatched; selection of tab 2134 presents a list of ordered products that have been shipped from the supplier and are in transit; selection of tab 2136 presents a list of ordered products that have been delivered to the laboratory and selection of tab 2137 presents only those reagents placed on the watchlist that have been ordered. The inventory management module recognises that stock is required, and monitors stock orders in the system to determine whether stock is expected to be delivered in required timeframe or needs to be followed up or reordered. In each tab, the number of orders placed for a product as well as e.g. the number of units of that product requested in each order can be shown, as well as an estimated delivery time/date. Progress symbol 2138 may be used to indicate visually how far through the order process a particular product order is.

Information presented to user 1100 by inventory management module 2100 is obtained by the module causing the device processor 1210 communicate with one or more instruments 1500 in the laboratory to receive or interrogate for inventory data for individual instruments, or for a plurality of instruments in the laboratory. This enables inventory management module 2100 to ascertain aggregated inventory levels across a plurality of instruments and, in preferred embodiments, to estimate based on current inventory levels and specimen processing steps scheduled to be performed by the one or more instruments, a time at which supply of a consumable will become critically low or exhausted. This may be communicated to user 1100 by symbols 2127 and 2129. Alternatively/additionally 'push' communication can be created by the inventory management module 2100 and delivered to the device display 1220 to alert the user 1100 that there is a critically low level of a particular consumable and prompting the user to take appropriate action such as attending the laboratory to refill or re-stock an instrument or reschedule one or more tests to avoid instrument downtime.

Ordering of one or more consumables for which there is no/low stock may be automated by inventory management module 2100, based on user-configurable ordering rules. Optionally, the rules may include prior finance approval from an account manager for a supplier of products used by the instruments, in which case invoicing to the laboratory may be automated.

The inventory management module 2100 utilises a location service, such as a geolocation based on a WiFi connection, GPS, or assisted GPS (cellular location system) module 1230 in the user device 1200 to determine automatically when the user device 1200 is in close physical proximity to an instrument 1500 in the laboratory that has an inventory management issue (e.g. low/no stock) and to issue automatically a 'push' notification to the device display 1220 advising the operator to perform an inventory management operation (such as re-filling/restocking a reagent on board an instrument) while they are conveniently close to the problematic instrument.

Preferably, user 1100 can set rules for push communications which need not be limited to proximity to an instrument 1500. Push notifications may also be permitted e.g. to communicate when a reagent has reached a critically low level and/or to communicate how many tests may be performed based on current inventory, and suggesting that the user place an order through the inventory management module 2100. Push notifications may also be used to communicate order status information to a user. Although referred to herein as a 'push' notification, it is to be understood that such notifications may be by way of a message on device display 1220, and/or SMS and/or email or using a range of other communications platforms with which the inventory management module 2100 may integrate.

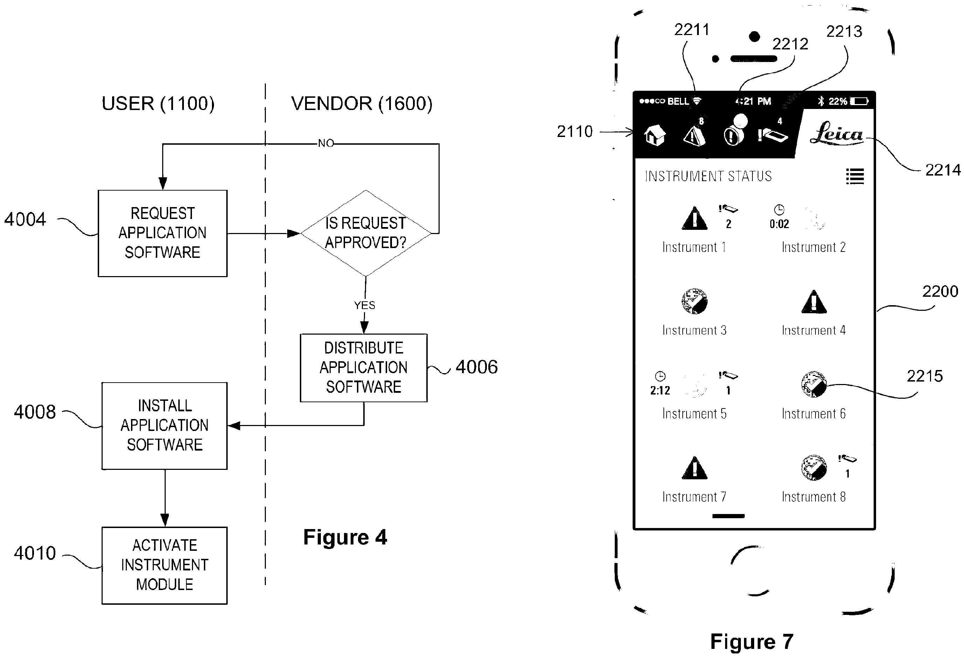

An instrument management module 2200 is configurable for customised management of one or more instruments 1500 in a laboratory. Figure 7 is an example of a display interface for a user device 1200 which has several display zones for providing instrument management information to the user. The instrument management module provides a user with a quick overview of the performance of a plurality of instruments 1500. Menu zone 2210 includes icons that are selectable by a user to quickly find instruments with e.g. urgent specimens to process, warnings or other cautions. By way of example, warning icon 2211 is ideally shown with a number (e.g. 8) indicating the number of instruments for which a warning is current e.g. for instruments with exhausted reagent or in an error condition. Caution icon 2212 is shown with a number (e.g. 5) indicating the number of instruments for which a caution message is current, e.g. for instruments with low reserves of reagent or requiring a service call in a short amount of time. Slide icon 2213 is shown with a number (e.g. 4) indicating the number of instruments that contain slides with specimens for urgent or priority processing. Selection of any one of these icons enables the user 1100 to drill down to the instrument level to ascertain more information about the warning, caution, or slides and the instrument/s to which they pertain. "Leica" icon 2214 is equivalent to a "home" icon and when selected, provides a dashboard-style overview, as shown in Figure 7. Here, the remaining area of the display is divided into 8 zones, each relating to a different instrument. Each instrument zone is configurable to provide one or more visual indicators relevant to the respective instrument. Major indicators may comprise e.g. icons such as those discussed above to identify instruments for which there are operational warnings (Instruments 1, 4 and 7), cautions (Instruments 2 and 5). A "functioning" icon 2215 indicates those instruments for which there are no warnings or cautions (Instruments 3, 6 and 8). Minor indicators may be included in each instrument zone such as e.g. time indicators to designate estimated time before the instrument will enter an error state or time at which the instrument is expected to finish processing a slide or a tray of slides. Urgent slide icon 2213 may be included in an instrument zone as a minor indicator to designate instruments that have urgent slides to process (Instruments 1, 5 and 8). Furthermore, icons representing nonactionable alarms or alarms that user cannot do anything about may be hidden from view.

Instrument management module 2200 may be configured to interrogate instruments for and/or receive automatically, status information, alert data, urgent specimen signals and the like. Module 2200 may also receive from user 1100 customisation rules for one or more of the instruments, for customisation of the instrument management module. The customisation rules may include but are not limited to user-defined instrument naming, user-defined instrument positioning on the device display, user-defined instrument parameters for presentation on the device display, and user-defined instrument control features.

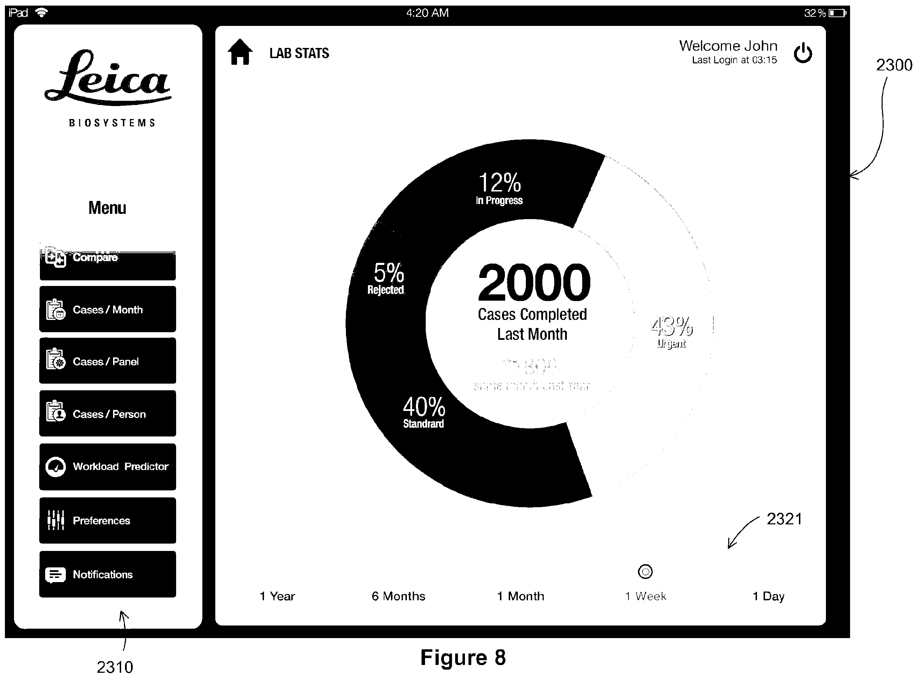

Statistics module 2300 may be configured to calculate and display statistics pertaining to usage of instruments 1500 in the laboratory. Statistics may be user-definable, or there may be a standard statistics suite in the module. The statistics module provides an overview to a user based on summary statistics of variables and data collected by the laboratory instruments as part of a usual archiving process. Sample case statistics are shown in Figure 8. Here, the total number of cases completed in a time period is shown as 2000 together with a comparison with the same period for the previous year, being 1800. The time period for which statistics are presented may be modified by selection of a time period shown at 2321 (e.g. 1 year, 6 months, 1 month, 1 week, 1 day). The slides (cases) for which statistics are presented may be grouped as e.g. in progress, urgent, standard and rejected.

Menu zone 2310 provides other options for viewing performance statistics relating to e.g. cases processed in a particular month, cases processed for a particular panel, (e.g. an antibody panel, a diagnostic panel, a breast cancer, or the like), cases processed for a particular pathologist, cases performed by a particular operator, for example a histotechnologist and a workload/predictor tool for projecting the number of cases likely to be processed in a particular future time period, based on current orders and/or historical statistics. This can be utilised by user 1100 (who may be e.g. a laboratory manager) to inform workforce management decisions and ensure adequate staffing (whilst minimising overstaffing) for operation of instruments 1500. The user may customise the statistical reporting by electing and modifying preferences.

A workflow module 2400 may be configured for customised management of specimen processing steps performed by one or more instruments in the laboratory. One feature of workflow module 2400 is a facility to receive, via a communications infrastructure, test orders placed by one or more pathologists who have sent a specimen to the laboratory for testing. The test request may be received using a schema that is automatically scheduled into an appropriate instrument 1500 by the workflow module e.g. where the pathologist has access to a "test request" via an online portal that uploads the request to the system 1000 via a communication infrastructure. Alternatively, the pathologist may request tests using electronic forms such as SMS, email, web-based form or the like that are automatically uploaded to the workflow module 2400 for manual scheduling by the user 1100. The workflow module 2400 also presents current status information for specimen processing steps requested using the workflow module.

The workflow module 2400 may also be configured to receive data pertaining to a specimen already processed on one of the instruments 1500 and automatically present to user 1200 a notification that results are available. Optionally, an image of the specimen or the results themselves, may be presented on device display 1220. In some embodiments, workflow module 2400 is configured to receive specimen data from one or more instruments 1500 and automatically present on the device display 1220 a recommended action (e.g. for further processing, an additional protocol, troubleshooting, suggestion to refer to website or information portal or the like). In some embodiments, workflow module 2400 is configured to receive specimen processing schedules from a plurality of instruments 1500 in the laboratory and automatically re-schedule one or more processing steps for one or more of the instruments to optimise one or more constraints. The constraints may include, but are not limited to, one or more of scheduled completion time, amount of consumables used, operational costs, staffing requirements and specimen processing value to the laboratory (e.g. financial versus reputational).

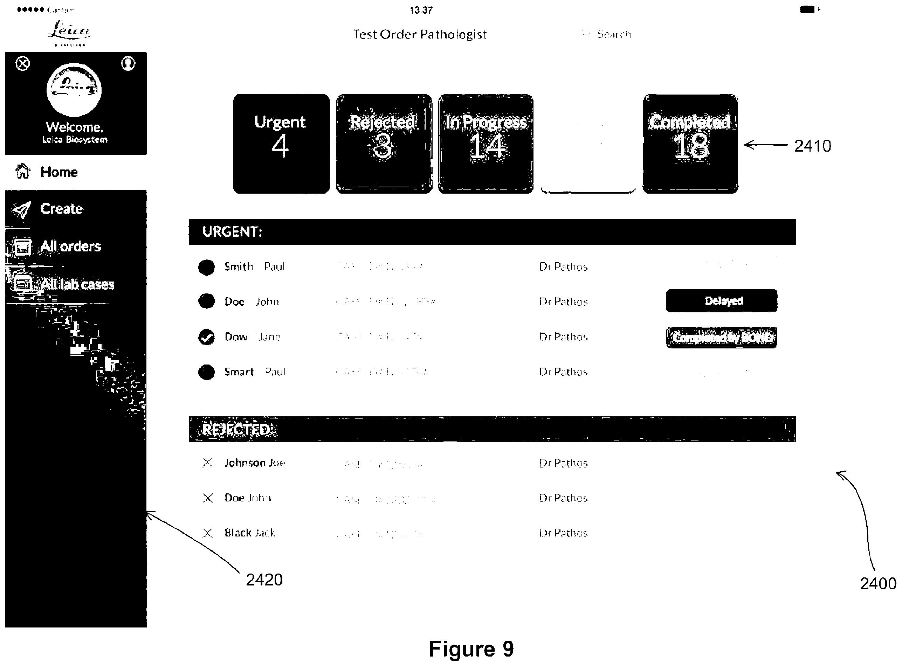

Figure 9 is an example of test information that may be made available by workflow module 2400. Summary zone 2410 includes tiles showing the tests ordered by a pathologist, grouped by those that are urgent, rejected, in progress, stained and completed. Selecting a tile in summary zone 2410 provides details of tests summarised in that tile. Figure 9 shows that the pathologist has requested four "urgent" tests. Selection of the "urgent" tile brings into view (as shown in Figure 9) details of those urgent tests, including patient name (optionally de-identified), case ID identifying the slide carrying the patient's specimen, name of the laboratory or responsible pathologist and status of the test. A menu zone 2420 lists options selectable by a clinician (e.g. using their own user device onto which the workflow module 2400 has been installed) to "create" a new test (e.g. for a specimen already identified in the system 1000), view all orders requested by the clinician, and view all cases requested by the pathologist's laboratory.

A maintenance module 2500 is configured for customised instrument maintenance functions and reporting. This may include interrogating and/or receiving automatically maintenance data from one or more instruments1500 in the laboratory and prompting a user 1100 by notification or a message on device display 1220 when ones of the instruments have a scheduled maintenance event due or approaching. A calendar feature may present visually when ones of the instruments are due for a service call or maintenance event and the user 1100 may request re-scheduling by the maintenance module to avoid or minimise instrument downtime and/or scheduling conflicts e.g. with urgent slide processing requests. Thus, maintenance module 2500 may be configured to interrogate for, receive or generate aggregated maintenance data from a plurality of laboratories, and revise a scheduled maintenance event based on the aggregated data or create a new maintenance schedule which optimises one or more constraints that may include e.g. scheduled instrument downtime, technician availability, impact on scheduled or expected specimen processing and the like.

A reporting module 2600 may be configured for customised reporting of instrument related performance data. This may include receiving at the user device 1200 user-defined specifications for data analysis and/or reporting. Reporting module 2600 may aggregate instrument performance data for a plurality of instruments 1500 in the laboratory and present on the device display 1200 laboratory performance information based on the aggregated instrument performance data. Reporting module 2600 may also cause transmission of laboratory and/or instrument specific performance data to a third party 1650 for aggregation with corresponding data from one or more other laboratories.

Aggregation of data by a third party 1650 can relate to any data that is collected or created by the instrument module 2000, or by instrument processors 1560. Aggregated data may be used to monitor across multiple sites (and multiple laboratories, potentially in multiple countries): laboratory performance and patterns in instrument usage, consumption of reagent and other consumables, popularity of tests, common maintenance issues and other information that may be useful to a third party. Aggregated data may be used to model performance and improve maintenance schedules, lead to proactive maintenance activities anticipating e.g. component wear on instruments that have not yet been used to the same extent as other instruments that have encountered a wear error and many other advantages are contemplated.

The third party 1650 may be a back-office associated with the proprietor of the system 1000 and/or instrument modules 2000 and may perform license management functions for software applications 1400 distributed by the system 1000. Typically, system 1000 is implemented in a "cloud computing" environment, although interface module 1300 is ideally provided in hardware, embedded in the laboratory in which the instruments 1500 are located. The cloud environment enables additional functionality to be added to instrument modules 2000 by wireless deployment of software patches on a "push" or "pull" basis, and upgrades of features over time. Additionally, software patches and additional functionality for one or more instruments 1500 may be purchased using an instrument module 2000 and, once unlocked, deployed from servers 1700 to the instruments via interface module 1300.

The functionality may further include asset tracking of instruments and customers that are using those instruments by instrument vendors. Any technical issues pertaining to an instrument may be directed to the instrument vendor together with user information, instrument identification and logged data, such that the instrument vendor can triage the instrument before a service call is requested.

It is to be understood that various modifications, additions and/or alterations may be made to the parts previously described without departing from the ambit of the present invention as defined in the claims appended hereto.

## Claims

1. A system (1000) for customising management of one or more instruments (1500a-d) in a laboratory, the system including:
(a) a user device (1200) adapted to receive inputs from a user (1100), the user device (1200) having a device processor (1210) for processing the received inputs and instrument data from the one or more instruments (1500a-d), and a device display (1220) for displaying instrument information;
(b) an interface module (2700) communicatively coupled with the user device (1200) and the one or more instruments (1500a-d) and configured to convert instrument data in a first format (1590) generated by an instrument processor (1510) of the one or more instruments (1500a-d) to a second format (1290) for processing by the device processor (1210); and
(c) an instrument module (2000) configured to receive the instrument data in the second format (1290) and configured to cause the device processor (1210) to provide customised instrument information on the device display (1220) and to perform processing functions that provide the user with a customised environment for interacting with the one or more instruments (1500a-d), wherein the instrument module (2000) is selectable from a suite of instrument modules with which the user device (1200) may be configured, the suite of modules including an inventory management module (2100); wherein the system further includes the inventory management module (2100) configured for customised management of inventory, which inventory management module (2100) is configured to:
(d) by means of a location service in the user device (1200), determine automatically when the user device (1200) is in close physical proximity to an instrument (1500) in the laboratory that has an inventory management issue; and to
(e) automatically generate a notification which it issues to the user device (1200) while in close proximity, identifying the instrument (1500) for which the user is required to perform an inventory management operation.

2. A system (1000) according to claim 1, wherein the user device (1200) is configurable with an instrument module (2000) by:
a) a user (1100) requesting application software (1400) corresponding to the instrument module (2000) from a remotely located vendor (1600);
b) the vendor (1600) approving the request and operating a distribution software program to deliver the requested application software (1400) to the user device (1200); and
c) the user (1100) installing the application software (1400) on the user device (1200) to activate the instrument module (2000).

3. A system (1000) according to any one of the preceding claims, the suite of modules further including:
(a) an instrument management module (2200)
(b) a statistics module (2300);
(c) a workflow module (2400);
(d) a maintenance module (2500); and
(e) a reporting module (2600).

4. A system (1000) according to any one of claims 1 to 3, wherein the inventory management module (2100) is configurable for customised inventory management by performing functions on the device processor (1210) selected from the group including:
(a) interrogating for and/or receiving automatically from one or more instruments (1500a-d) an inventory level for individual ones of the instruments (1500a-d);
(b) interrogating for and/or receiving automatically from instruments (1500a-d) in the laboratory aggregated inventory levels across a plurality of instruments (1500a-d) in the laboratory;
(c) interrogating for and/or receiving automatically from one or more instruments (1500a-d) in the laboratory an estimated time at which supply of a consumable in the laboratory will be critically low;
(d) automatic ordering of one or more consumables used by an instrument (1500a-d) in the laboratory;
(e) tracking order status for consumables ordered by the inventory management module (2100);
(f) presenting on the device display (1220) inventory management information; and
(g) automatically invoicing an entity for consumables ordered by the inventory management module (2100).

5. A system (1000) according to claim 3, wherein the instrument management module (2000) is configurable for customised instrument management by performing functions on the device processor (1210) selected from the group including:
(a) interrogating for and/or receiving automatically instrument status information from one or more instruments (1500a-d) and presenting on the device display (1220) status information for the one or more instruments (1500a-d), wherein the instrument status is preferably represented by an icon;
(b) interrogating for and/or receiving automatically alert data from one or more instruments(1500a-d) and presenting on the device display (1220) an alert symbol or notification, for respective ones of the instruments (1500);
(c) interrogating for and/or receiving automatically an urgent specimen signal and presenting on the device display (1220) an urgency symbol or notification for one or more of the instruments (1500a-d) associated with the urgent specimen signal;
(d) receiving from the user customisation rules, and customising the instrument management module (2000) according to the customisation rules.

6. A system (1000) according to claim 6, wherein the customisation rules are selected from a group including:
(a) user-defined instrument naming;
(b) user-defined instrument positioning on the display (1220);
(c) user-defined instrument parameters for presentation on the display (1220); and
(d) user-defined instrument control features controllable by the user device (1200) using the instrument management module (2000).

7. A system (1000) according to claim 3, wherein the workflow module (2400) is configured for customised management of specimen processing steps performed by one or more instruments (1500a-d) in the laboratory, by performing functions on the device processor (1210) selected from the group including:
(a) receiving electronically a request for a specimen processing step not already scheduled on an instrument (1500) and causing scheduling of the requested processing step on the instrument (1500);
(b) receiving specimen data from one or more instruments (1500a-d) and causing the user device (1200) to display a related image or processing step result on the display (1220);
(c) processing specimen data received from one or more instruments (1500a-d) and automatically presenting on the device display (1220) a recommended action;
(d) receiving specimen processing schedules from a plurality of instruments (1500a-d) and automatically re-scheduling one or more processing steps to optimise one or more constraints selected from the group including scheduled completion time, consumables used, operational cost, staffing requirements and specimen processing value; and
(e) presenting current status information for specimen processing steps.

8. A system (1000) according to claim 3, wherein the maintenance module (2500) is configurable for customised instrument maintenance by performing functions on the device processor (1210) selected from the group including:
(a) interrogating for and/or receiving automatically maintenance data from one or more instruments (1500a-d) in the laboratory and causing the user device (1200) to display a prompt when ones of the instruments (1500a-d) have a scheduled maintenance event;
(b) interrogating and/or receiving automatically aggregated maintenance data from a plurality of laboratories and adjusting a scheduled maintenance event based on the aggregated data;
(c) aggregating scheduled maintenance events for a plurality of instruments (1500a-d) in the laboratory and creating new maintenance schedule which optimises one or more constraints selected from the group including scheduled instrument downtime, technician availability, and impact on scheduled or expected specimen processing; and
(d) transmitting to a remote processing device maintenance data from the one or more instruments (1500a-d) for aggregation with maintenance data from one or more other laboratories.

9. A system (1000) according to claim 3, wherein the reporting module (2600) is configurable for customised reporting of instrument related performance data, by performing functions on the device processor (1210) selected from the group including:
(a) receiving operator defined specifications for data analysis;
(b) receiving operator defined specifications for data reporting;
(c) aggregating instrument performance data for a plurality of instruments (1500a-d) in the laboratory and presenting on the device display (1220) laboratory performance information based on the aggregated instrument performance data;
(d) transmitting to a remote processing device instrument and/or laboratory specific performance data for aggregation with performance data from one or more other laboratories.

10. A system (1000) according to any one of the preceding claims, wherein the user device (1200) is a device selected from the group including a smart phone, tablet computer, notebook computer, netbook computer, body-worn computer and other mobile computing devices.

11. A system (1000) according to any one of the preceding claims, wherein the instrument module (2000) is configurable to determine automatically when the user device (1200) is in close physical proximity to an instrument (1500) in the laboratory; and automatically issue a notification to the user device (1200) which is relevant to the instrument (1500) in close proximity.

12. A system (1000) according to any one of the preceding claims, wherein the instrument module (2000) is configurable in accordance with customisation rules received from the user (1100) as inputs to the user device (1200).

## Patentansprüche

1. System (1000) zur kundenspezifischen Verwaltung von einem oder mehreren Instrumenten (1500a-d) in einem Labor, wobei das System umfasst:
(a) eine Benutzervorrichtung bzw. -gerät (1200), die dazu eingerichtet ist, Eingaben von einem Benutzer (1100) zu empfangen, wobei die Benutzervorrichtung (1200) einen Geräteprozessor (1210) zum Verarbeiten der empfangenen Eingaben und Gerätedaten von dem einen oder den mehreren Instrumenten (1500a-d) und eine Geräteanzeige (1220) zum Anzeigen von Instrumenteninformationen aufweist;
(b) ein Schnittstellenmodul (2700), das kommunikativ mit dem Benutzergerät (1200) und dem einen oder den mehreren Instrumenten (1500a-d) gekoppelt und dazu eingerichtet ist, Instrumentendaten in einem ersten Format (1590), das von einem Instrumentenprozessor (1510) des einen oder der mehreren Instrumente (1500a-d) erzeugt wird, in ein zweites Format (1290) zur Verarbeitung durch den Geräteprozessor (1210) umzuwandeln; und
(c) ein Instrumentenmodul (2000), das dazu eingerichtet ist, die Instrumentendaten in dem zweiten Format (1290) zu empfangen, und dazu eingerichtet ist, den Geräteprozessor (1210) zu veranlassen, angepasste Instrumenteninformationen auf der Geräteanzeige (1220) bereitzustellen und Verarbeitungsfunktionen auszuführen, die dem Benutzer eine angepasste Umgebung für die Interaktion mit dem einen oder den mehreren Instrumenten (1500a-d) bereitstellen, wobei das Instrumentenmodul (2000) aus einer Reihe von Instrumentenmodulen auswählbar ist, mit denen das Benutzergerät (1200) eingerichtet werden kann, wobei die Reihe von Modulen ein Bestandsverwaltungsmodul (2100) enthält; wobei das System ferner das Bestandsverwaltungsmodul (2100) enthält, das für eine kundenspezifische Verwaltung des Bestands eingerichtet ist, wobei das Bestandsverwaltungsmodul (2100) dazu eingerichtet ist, :
(d) mittels eines Ortungsdienstes in der Benutzervorrichtung (1200) automatisch zu bestimmen, wenn sich die Benutzervorrichtung (1200) in unmittelbarer physischer Nähe zu einem Instrument (1500) in dem Labor befindet, das ein Bestandsverwaltungsproblem hat; und
(e) automatisch eine Benachrichtigung zu erzeugen, die es an die Benutzervorrichtung (1200) ausgibt, während sie sich in unmittelbarer Nähe befindet, und die das Instrument (1500) identifiziert, für das der Benutzer eine Bestandsverwaltungsoperation durchführen muss.

2. System (1000) nach Anspruch 1, wobei das Benutzergerät (1200) mit einem Instrumentenmodul (2000) einrichtbar ist durch
a) einen Benutzer (1100), der Anwendungssoftware (1400), die dem Instrumentenmodul (2000) entspricht, von einem entfernt gelegenen Anbieter (1600) anfordert;
b) den Anbieter (1600), der die Anforderung genehmigt und ein Verteilungssoftwareprogramm betreibt, um die angeforderte Anwendungssoftware (1400) an das Benutzergerät (1200) zu liefern; und
c) den Benutzer (1100), der die Anwendungssoftware (1400) auf dem Benutzergerät (1200) installiert, um das Instrumentenmodul (2000) zu aktivieren.

3. System (1000) nach einem der vorhergehenden Ansprüche, wobei die Reihe von Modulen ferner umfasst:
(a) ein Instrumentenverwaltungsmodul (2200);
(b) ein Statistikmodul (2300);
(c) ein Workflow-Modul (2400);
(d) ein Wartungsmodul (2500); und
(e) ein Berichtsmodul (2600).

4. System (1000) nach einem der Ansprüche 1 bis 3, wobei das Bestandsverwaltungsmodul (2100) für eine kundenspezifische Bestandsverwaltung eingerichtet ist, indem es Funktionen auf dem Geräteprozessor (1210) durchführt, die aus der Gruppe ausgewählt sind, die Folgendes umfasst:
(a) Abfragen und/oder automatisches Empfangen von einem oder mehreren Instrumenten (1500a-d) eines Bestandsniveaus für einzelne der Instrumente (1500a-d);
(b) Abfragen und/oder automatisches Empfangen von Instrumenten (1500a-d) im Labor von aggregierten Bestandsniveaus über eine Vielzahl von Instrumenten (1500a-d) im Labor;
(c) Abfragen und/oder automatisches Empfangen von einem oder mehreren Instrumenten (1500a-d) im Labor eines geschätzten Zeitpunkts, zu dem der Vorrat eines Verbrauchsmaterials im Labor kritisch niedrig sein wird;
(d) automatisches Bestellen von einem oder mehreren Verbrauchsmaterialien, die von einem Instrument (1500a-d) im Labor verwendet werden;
(e) Verfolgen des Bestellstatus für Verbrauchsmaterialien, die vom Bestandsverwaltungsmodul (2100) bestellt wurden
(f) Darstellen von Bestandsverwaltungsinformationen auf dem Gerätedisplay (1220); und
(g) automatisches In-Rechnung-Stellen einer Entität für Verbrauchsmaterialien, die vom Bestandsverwaltungsmodul (2100) bestellt wurden.

5. System (1000) nach Anspruch 3, wobei das Instrumentenmanagementmodul (2000) für ein kundenspezifisches Instrumentenmanagement eingerichtet ist, indem es Funktionen auf dem Geräteprozessor (1210) durchführt, die aus der Gruppe ausgewählt sind, die Folgendes umfasst:
(a) Abfragen und/oder automatisches Empfangen von Instrumentenstatusinformationen von einem oder mehreren Instrumenten (1500a-d) und Darstellen von Statusinformationen für das eine oder die mehreren Instrumente (1500a-d) auf dem Gerätedisplay (1220), wobei der Instrumentenstatus vorzugsweise durch ein Symbol dargestellt wird;
(b) Abfragen und/oder automatisches Empfangen von Alarmdaten von einem oder mehreren Instrumenten (1500a-d) und Darstellen eines Alarmsymbols oder einer Benachrichtigung auf dem Gerätedisplay (1220) für entsprechende Instrumente (1500);
(c) Abfragen und/oder automatisches Empfangen eines dringenden Probensignals und Darstellen eines Dringlichkeitssymbols oder einer Benachrichtigung auf dem Gerätedisplay (1220) für eines oder mehrere der Instrumente (1500a-d), die mit dem dringenden Probensignal assoziiert sind;
(d) Empfangen von Anpassungsregeln vom Benutzer und Anpassen des Instrumentenverwaltungsmoduls(2000) gemäß den Anpassungsregeln.

6. System (1000) nach Anspruch 6, wobei die Anpassungsregeln aus einer Gruppe ausgewählt sind, die Folgendes umfasst:
(a) benutzerdefinierte Instrumentenbenennung;
(b) benutzerdefinierte Instrumentenpositionierung auf dem Display (1220);
(c) benutzerdefinierte Instrumentenparameter zur Darstellung auf dem Display (1220); und
(d) benutzerdefinierte Instrumentensteuerungsmerkmale, die von der Benutzervorrichtung (1200) unter Verwendung des Instrumentenverwaltungsmoduls (2000) steuerbar sind.

7. System (1000) nach Anspruch 3, wobei das Arbeitsablauf- bzw. Workflowmodul (2400) dazu eingerichtet ist, die von einem oder mehreren Instrumenten (1500a-d) im Labor durchgeführten Probenverarbeitungsschritte individuell zu verwalten, indem es auf dem Geräteprozessor (1210) Funktionen ausführt, die aus der Gruppe ausgewählt sind, die Folgendes umfasst
(a) elektronischer Empfang einer Anforderung für einen Probenverarbeitungsschritt, der noch nicht auf einem Instrument (1500) eingeplant ist, und Veranlassung der Einplanung des angeforderten Verarbeitungsschritts auf dem Instrument (1500);
(b) Empfangen von Probendaten von einem odermehreren Instrumenten (1500a-d) und Veranlassen, dass das Benutzergerät (1200) ein zugehöriges Bild oder ein Verarbeitungsschritt-Ergebnis auf dem Display (1220) anzeigt;
(c) Verarbeiten von Probendaten, die von einem oder mehreren Instrumenten (1500a-d) empfangen wurden und automatisches Darstellen einer empfohlenen Aktion auf dem Gerätedisplay (1220);
(d) Empfangen von Probenverarbeitungsplänen von einer Vielzahl von Instrumenten (1500a-d) und automatisches Neuplanen eines oder mehrerer Verarbeitungsschritte, um eine oder mehrere Beschränkungen zu optimieren, die aus der Gruppe ausgewählt werden, die die geplante Fertigstellungszeit, die verwendeten Verbrauchsmaterialien, die Betriebskosten, die Personalanforderungen und den Probenverarbeitungswert umfasst; und
(e) Darstellen von aktuellen Statusinformationen für Probenverarbeitungsschritte.

8. System (1000) nach Anspruch 3, wobei das Wartungsmodul (2500) für eine kundenspezifische Instrumentenwartung eingerichtet ist, indem es Funktionen auf dem Geräteprozessor (1210) durchführt, die aus der Gruppe ausgewählt sind, die Folgendes umfasst:
(a) Abfragen und/oder automatisches Empfangen von Wartungsdaten von einem oder mehreren Instrumenten (1500a-d) im Labor und Bewirken, dass die Benutzervorrichtung (1200) eine Eingabeaufforderung anzeigt, wenn eines der Instrumente (1500a-d) ein geplantes Wartungsereignis hat;
(b) Abfragen und/oder automatisches Empfangen von aggregierten Wartungsdaten von einer Vielzahl von Laboren und Anpassen eines geplanten Wartungsereignisses auf der Grundlage der aggregierten Daten;
(c) Aggregieren geplanter Wartungsereignisse für eine Vielzahl von Instrumenten (1500a-d) in dem Labor und Erstellen eines neuen Wartungsplans, der eine oder mehrere Beschränkungen optimiert, die aus der Gruppe ausgewählt werden, die geplante Instrumentenausfallzeiten, Technikerverfügbarkeit und Auswirkungen auf die geplante oder erwartete Probenverarbeitung umfasst; und
(d) Übertragen von Wartungsdaten von dem einen oder den mehreren Instrumenten (1500a-d) an eine entfernte Verarbeitungsvorrichtung zur Aggregation mit Wartungsdaten von einem oder mehreren anderen Laboren.

9. System (1000) nach Anspruch 3, wobei das Melde- bzw. Berichtsmodul (2600) für eine kundenspezifische Meldung von instrumentenbezogenen Leistungsdaten eingerichtet ist, indem es Funktionen auf dem Geräteprozessor (1210) durchführt, die aus der Gruppe ausgewählt sind, die Folgendes umfasst:
(a) Empfangen von durch den Bediener definierten Spezifikationen für die Datenanalyse;
(b) Empfangen von durch den Bediener definierten Spezifikationen für die Datenberichterstattung;
(c) Aggregieren von Instrumentenleistungsdaten für eine Vielzahl von Instrumenten (1500a-d) im Labor und Darstellen von Laborleistungsinformationen auf dem Gerätedisplay (1220) auf der Grundlage der aggregierten Instrumentenleistungsdaten;
(d) Übertragen von instrumenten- und/oder laborspezifischen Leistungsdaten an eine entfernte Verarbeitungsvorrichtung zur Aggregation mit Leistungsdaten von einem oder mehreren anderen Laboren.

10. System (1000) nach einem der vorhergehenden Ansprüche, wobei das Benutzergerät (1200) ein Gerät ist, das aus der Gruppe ausgewählt ist, die ein Smartphone, einen Tablet-Computer, einen Notebook-Computer, einen Netbook-Computer, einen am Körper getragenen Computer und andere mobile Computergeräte umfasst.

11. System (1000) nach einem der vorhergehenden Ansprüche, wobei das Gerätemodul (2000) dazu eingerichtet ist, automatisch festzustellen, wann sich das Benutzergerät (1200) in unmittelbarer physischer Nähe zu einem Instrument (1500) im Labor befindet, und automatisch eine Benachrichtigung an das Benutzergerät (1200) auszugeben, die für das Instrument (1500) in unmittelbarer Nähe relevant ist.

12. System (1000) nach einem der vorhergehenden Ansprüche, wobei das Gerätemodul (2000) in Übereinstimmung mit Anpassungsregeln, die vom Benutzer (1100) als Eingaben in das Benutzergerät (1200) empfangen werden, einrichtbar ist.

## Revendications

1. Système (1000) pour personnaliser la gestion d'un ou plusieurs instruments (1500a-d)dans un laboratoire, le système comprenant :
(a) un dispositif utilisateur (1200) adapté pour recevoir des entrées d'un utilisateur (1100), le dispositif utilisateur (1200) ayant un processeur de dispositif (1210) pour traiter les entrées reçues et les données d'instrument provenant d'un ou plusieurs instruments (1500a-d), et un écran de dispositif (1220) pour afficher des informations sur les instruments ;
(b) un module d'interface (2700) couplé de manière communicative avec le dispositif utilisateur (1200) et le ou les instruments (1500a-d) et configuré pour convertir les données d'instrument dans un premier format (1590) généré par un processeur d'instrument (1510) du ou des instruments (1500a-d) en un deuxième format (1290) pour une opération par le processeur de dispositif (1210); et
(c) un module d'instrument (2000) configuré pour recevoir les données d'instrument dans le deuxième format (1290) et configuré pour amener le processeur de dispositif (1210) à fournir des informations d'instrument personnalisées sur l'écran du dispositif (1220) et à exécuter des fonctions de traitement qui fournissent à l'utilisateur un environnement personnalisé pour interagir avec l'un ou plusieurs instruments (1500a-d), dans lequel le module d'instrument (2000) peut être sélectionné dans une suite de modules d'instrument avec lesquels le dispositif d'utilisateur (1200) peut être configuré, la suite de modules comprenant un module de gestion de l'inventaire (2100) ; dans lequel le système comprend en outre le module de gestion d'inventaire (2100) configuré pour une gestion personnalisée de l'inventaire, lequel module de gestion d'inventaire (2100) est configuré pour :
(d) au moyen d'un service de localisation dans le dispositif de l'utilisateur (1200), déterminer automatiquement quand le dispositif de l'utilisateur (1200) est à proximité physique d'un instrument (1500) dans le laboratoire qui a un problème de gestion d'inventaire ; et pour
(e) générer automatiquement une notification qu'il émet au dispositif de l'utilisateur (1200) lorsqu'il est à proximité, identifiant l'instrument (1500) pour lequel l'utilisateur est tenu d'effectuer une opération de gestion d'inventaire.

2. Système (1000) selon la revendication 1, dans lequel le dispositif utilisateur (1200) est configurable avec un module d'instrument (2000) par
a) un utilisateur (1100) demandant un logiciel d'application (1400) correspondant au module d'instrument (2000) à un vendeur (1600) situé à distance
b) le vendeur (1600) approuvant la demande et opérant un programme logiciel de distribution pour livrer le logiciel d'application (1400) demandé au dispositif utilisateur (1200) et
c) l'utilisateur(1100) installant le logiciel d'application (1400) sur le dispositif utilisateur (1200) pour activer le module d'instrument (2000).

3. Système (1000) selon l'une quelconque des revendications précédentes, la suite de modules comprenant en outre
(a) un module de gestion des instruments (2200) ;
(b) un module de statistiques (2300) ;
(c) un module de flux de travail (2400) ;
(d) un module de maintenance (2500) ; et
(e) un module de rapport (2600).

4. Système (1000) selon l'une quelconque des revendications 1 à 3, dans lequel le module de gestion des stocks ou de gestion d'inventaire (2100) est configurable pour une gestion personnalisée des stocks en exécutant des fonctions sur le processeur du dispositif (1210) choisies dans le groupe comprenant :
(a) interrogeant et/ou recevant automatiquement d'un ou de plusieurs instruments (1500a-d) un niveau d'inventaire pour chacun des instruments (1500a-d) ;
(b) interrogeant et/ou recevant automatiquement d'instruments (1500a-d) dans le laboratoire des niveaux d'inventaire agrégés pour une pluralité d'instruments (1500a-d) dans le laboratoire ;
(c) interrogeant et/ou recevant automatiquement d'un ou de plusieurs instruments (1500a-d) dans le laboratoire une estimation du moment où l'approvisionnement d'un consommable dans le laboratoire sera critiquement bas ;
(d) commander automatiquement un ou plusieurs consommables utilisés par un instrument (1500a-d) dans le laboratoire ;
(e) tracer l'état des commandes de consommables commandés par le module de gestion des stocks (2100) ;
(f) présenter sur l'écran du dispositif (1220) des informations sur la gestion des stocks ; et
(g) facturer automatiquement une entité pour des consommables commandés par le module de gestion des stocks (2100).

5. Système (1000) selon la revendication 3, dans lequel le module (2000) de gestion des instruments est configurable pour une gestion personnalisée des instruments en exécutant des fonctions sur le processeur (1210) du dispositif choisies dans le groupe comprenant :
(a) interrogeant et/ou recevant automatiquement des informations d'état d'instrument d'un ou plusieurs instruments (1500a-d) et présentant sur l'écran du dispositif (1220) des informations d'état pour un ou plusieurs instruments (1500ad), dans lequel l'état de l'instrument est de préférence représenté par une icône
(b) interrogeant et/ou recevant automatiquement des données d'alerte d'un ou plusieurs instruments(1500a-d) et présentant sur l'écran du dispositif (1220) un symbole d'alerte ou une notification, pour des instruments respectifs (1500);
(c) interroger et/ou recevoir automatiquement un signal d'échantillon urgent et présenter sur l'écran du dispositif (1220) un symbole ou une notification d'urgence pour un ou plusieurs des instruments (1500a-d) associés au signal d'échantillon urgent ;
(d) recevoir de l'utilisateur des règles de personnalisation, et personnaliser le module de gestion des instruments (2000) selon les règles de personnalisation.

6. Système (1000) selon la revendication 6, dans lequel les règles de personnalisation sont choisies dans un groupe comprenant :
(a) dénomination de l'instrument définie par l'utilisateur ;
(b) positionnement de l'instrument défini par l'utilisateur sur l'écran (1220) ;
(c) paramètres de l'instrument définis par l'utilisateur pour la présentation sur l'écran (1220) ; et
(d) caractéristiques de contrôle de l'instrument définies par l'utilisateur et contrôlables par le dispositif de l'utilisateur (1200) à l'aide du module de gestion de l'instrument (2000).

7. Système (1000) selon la revendication 3, dans lequel le module de flux de travail (2400) est configuré pour une gestion personnalisée des étapes de traitement des échantillons réalisées par un ou plusieurs instruments (1500a-d) dans le laboratoire, en exécutant des fonctions sur le processeur du dispositif (1210) choisies dans le groupe comprenant :
(a) recevoir électroniquement une demande pour une étape de traitement d'échantillons qui n'est pas déjà programmée sur un instrument(1500) et provoquer la programmation de l'étape de traitement demandée sur l'instrument (1500
(b) recevant des données de spécimen d'un ou de plusieurs instruments (1500ad) et amenant le dispositif utilisateur (1200) à afficher une image ou un résultat d'étape de traitement connexe sur l'écran (1220) ;
(c) traitant les données de spécimen reçues d'un ou de plusieurs instruments (1500a-d) et présentant automatiquement sur l'écran du dispositif (1220) action recommandée ;
(d) recevant les programmes de traitement des échantillons d'une pluralité d'instruments (1500a-d) et reprogrammant automatiquement une ou plusieurs étapes de traitement pour optimiser une ou plusieurs contraintes sélectionnées dans le groupe comprenant le temps d'achèvement prévu, les consommables utilisés, le coût opérationnel, les besoins en personnel et la valeur de traitement des échantillons ; et
(e) présentant des informations sur l'état actuel des étapes de traitement des échantillons.

8. Système (1000) selon la revendication 3, dans lequel le module de maintenance (2500) est configurable pour une maintenance personnalisée des instruments en exécutant des fonctions sur le processeur du dispositif (1210) choisies dans le groupe comprenant :
(a) interrogeant et/ou recevant automatiquement des données de maintenance d'un ou plusieurs instruments (1500a-d) dans le laboratoire et amenant le dispositif utilisateur (1200) à afficher une invite lorsque l'un des instruments (1500a-d) a un événement de maintenance programmé ;
(b) interrogeant et/ou recevant automatiquement des données de maintenance agrégées d'une pluralité de laboratoires et ajustant un événement de maintenance programmé sur la base des données agrégées ;
(c) agréger les événements de maintenance programmés pour une pluralité d'instruments (1500a-d) dans le laboratoire et créer un nouveau programme de maintenance qui optimise une ou plusieurs contraintes choisies dans le groupe comprenant le temps d'arrêt programmé de l'instrument, la disponibilité du technicien et l'impact sur le traitement programmé ou prévu des spécimens ; et
(d) transmettre à un dispositif de traitement à distance les données de maintenance d'un ou de plusieurs instruments (1500a-d) pour les agréger avec les données de maintenance d'un ou de plusieurs autres laboratoires.

9. Système (1000) selon la revendication 3, dans lequel le module de rapport (2600) est configurable pour un rapport personnalisé des données de performance liées à l'instrument, en exécutant des fonctions sur le processeur du dispositif (1210) choisies dans le groupe comprenant :
(a) recevoir les spécifications définies par l'opérateur pour l'analyse des données;
(b) recevoir les spécifications définies par l'opérateur pour le rapport des données;
(c) agréger les données de performance des instruments pour une pluralité d'instruments (1500a-d) dans le laboratoire et présenter sur l'écran du dispositif (1220) les informations de performance du laboratoire basées sur les données de performance des instruments agrégés ;
(d) transmettre à un dispositif de traitement à distance les données de performance spécifiques à l'instrument et/ou au laboratoire pour les agréger avec les données de performance d'un ou de plusieurs autres laboratoires.

10. Système (1000) selon l'une quelconque des revendications précédentes, dans lequel le dispositif utilisateur (1200) est un dispositif choisi dans le groupe comprenant un téléphone intelligent, un ordinateur tablette, un ordinateur portable, un ordinateur netbook, un ordinateur porté sur le corps et d'autres dispositifs informatiques mobiles.

11. Système (1000) selon l'une quelconque des revendications précédentes, dans lequel le module d'instrument (2000) est configurable pour déterminer automatiquement lorsque le dispositif d'utilisateur (1200) est à proximité physique d'un instrument (1500) dans le laboratoire ; et émettre automatiquement une notification au dispositif d'utilisateur (1200) qui est pertinente pour l'instrument (1500) à proximité.

12. Système (1000) selon l'une quelconque des revendications précédentes, dans lequel le module d'instrument (2000) est configurable conformément aux règles de personnalisation reçues de l'utilisateur (1100) en tant qu'entrées du dispositif utilisateur (1200).
